# EUROPÄISCHE PATENTANMELDUNG

(11) **EP 3 345 578 A1**
(43) Veröffentlichungstag der Anmeldung: **11.07.2018**
(21) Anmeldenummer: 18000090.3
(22) Anmeldetag: 03.01.2015
(51) Int. Cl.: A61F 5/01, A61F 5/055, A61B 17/02

(54) **BELEUCHTBARER MEDIZINISCHER RETRAKTOR**

(30) Priorität: 26.12.2013 DE 102013021857
(62) Teilanmeldung aus: 15700642.0
(71) Anmelder: Lang, Reinhold, 82064 Straßlach (DE)
(72) Erfinder: Lang, Reinhold, 82064 Straßlach (DE)

(57) **Zusammenfassung**

Ein Medizinischer Retraktor (1) weist einen Griffabschnitt (2), einen Hakenabschnitt (3) und einen Deckel (6) aufweist. Der Griffabschnitt (2) ist zumindest abschnittsweise als einen Hohlraum (4) aufweisenden Hohlkörper ausgebildet. Der Hakenabschnitt (3) weist wenigstens einen Lichtleiter (5) mit einer Lichteintrittsfläche und einer Lichtaustrittsfläche auf. Die Lichteintrittsfläche (51) des wenigstens einen Lichtleiters (5) ist in dem in dem Griffabschnitt (2) ausgebildeten Hohlraum (4) angeordnet, oder bündig zu einer Innenwand des in dem Griffabschnitt (2) ausgebildeten Hohlraums (4) angeordnet, oder so angeordnet, dass sie hin zu dem in dem Griffabschnitt (2) ausgebildeten Hohlraum (4) orientiert ist. Die Lichtaustrittsfläche (52) des wenigstens einen Lichtleiters (5) ist weg vom Griffabschnitt (2) orientiert. Der Deckel (6) verschließt den im Griffabschnitt (2) ausgebildeten Hohlraum (4) insbesondere flüssigkeitsdicht und weiter insbesondere luftdicht. Am Griffabschnitt (2) ist wenigstens ein Haken (15*) ausgebildet, der bei verschlossenem Deckel (6) mit dem Deckel (6) in Eingriff kommt. Dabei weist der Griffabschnitt wenigstens eine Sollbruchstelle (15") auf, welche durch Abbrechen des wenigstens einen Hakens (15*) ein Öffnen des verschlossenen Deckels erlaubt.

## Beschreibung

Die Erfindung betrifft einen beleuchteten medizinischer Retraktor und damit einen medizinischen Haltehaken, mit dessen Hilfe bei chirurgischen Eingriffen der Zugang zu einem Operationsfeld durch Gewebespreizung offengehalten oder ermöglicht wird.

Um einen möglichst ungestörten Zugang zu einem interessierenden Operationsfeld zu haben, ist es während chirurgischer Eingriffe häufig erforderlich, umliegendes Gewebe mittels eines Retraktor zurückzuhalten oder aufzuspreizen. Hierfür wird der Retraktor meist durch eine Fachpflegekraft im Operationsdienst (z.B. einen Operationstechnischen Assistenten, einen Chirurgisch-Technischen-Assistenten oder eine Operationspflegekraft) oder einen assistierenden Arzt gehalten. Wenn Retraktoren dauerhaft in einer konstanten Position gehalten werden müssen, ist es auch bekannt, diese für die Dauer eines chirurgischen Eingriffes an einem verwendeten Operationstisch zu befestigen.

Ein Retraktor ist häufig als im wesentlichen rechtwinkliger Körper aus Edelstahl ausgebildet, dessen einer Schenkel verrundet abgeflacht ist, und dessen anderer Schenkel verdickt als im wesentlichen zylindrischer Handgriff ausbildet ist.

Während eines chirurgischen Eingriffes ist eine gute Ausleuchtung des Operationsfeldes von großer Bedeutung. Dabei ist eine über das ganze Operationsfeld homogene und möglichst schattenfreie Beleuchtung mit hoher Beleuchtungsstärke wünschenswert. Für die Ausleuchtung des Operationsfeldes werden meist Operationsleuchten verwendet, die oberhalb eines Operationstisches an einem Deckenstativ angebracht sind.

Bei tief im Gewebe eines Patienten liegenden Operationsfeldern ist die gewünschte homogene und schattenfreie Ausleuchtung häufig nur schwer zu erreichen, da die Gefahr besteht, dass die Operationsleuchte durch einen behandelnden Arzt oder ein von dem behandelnden Arzt geführtes chirurgisches Instrument verdeckt wird. Diese Gefahr besteht insbesondere auch bei minimal-invasiven Eingriffen.

Aus der US 2007/0208226 ist bekannt, entlang der Oberseite eines Retraktors einen Lichtwellenleiter anzuordnen, der optisch mit einer im Bereich des Haltegriffs des Retraktors angeordneten Lichtquelle verbunden ist. Dabei ist die Befestigung des Lichtwellenleiters am Retraktor unbefriedigend gelöst. Zudem ist die Handhabung kompliziert, da für unterschiedliche Retraktoren unterschiedliche zugehörige Lichtwellenleiter benötigt werden. Schließlich besteht die Gefahr, dass sich der Lichtwellenleiter während eines chirurgischen Eingriffes löst und einen behandelnden Arzt behindert.

Aus der WO 2012/045459 A1 ist ein beleuchteter Haltehaken mit einem auswechselbaren Hakenelement aus einem Polycarbonat- oder Polymethylmethacrylat-Kunststoffmaterial hoher mechanischer Belastbarkeit sowie hoher Schlagzähigkeit bekannt, welches Hakenelement über eine formschlüssige Kupplung mit einem Frontende eines Handgriffs verbunden ist. Der Handgriff ist als Hohlkörper zur Aufnahme eines Energiespeichers ausgestaltet. In das dem Hakenelement zugekehrte Frontende des Handgriffs ist eine weißlichtemittierende Diode sowie deren Speiseschaltung eingesetzt. In das Hakenelement ist ein Lichtführungsstrang integriert, der einen gekrümmten Querschnitt aufweist und im Bereich seines freien Lichtaustrittsendes konisch spitz zulaufend mit verjüngter Lichtauskoppelfläche ausgebildet ist, die einen punktähnlichen Lichtaustritt gewährleistet.

Das Hakenelement ist als auswechselbares Einwegelement vorgesehen, so dass eine nachträgliche Reinigung und Sterilisation entfällt. Auch hier ist die zweiteilige Ausgestaltung des Haltehakens nachteilig. Da die gesamte Kraft über die Kupplung zwischen dem Haltehaken und dem Handgriff übertragen werden muss, besteht die Gefahr, dass sich der Handgriff von dem Haltehaken während eines chirurgischen Eingriffes löst und einen behandelnden Arzt behindert. Zudem kann nur ein sehr kleiner Teil des Operationsfeldes bestrahlt werden. Weiter muss der Handgriff nach jeder Verwendung sterilisiert werden, was die Lebenserwartung des Energiespeichers und der Diode stark verkürzt. Insbesondere die optischen Eigenschaften einer Lichtaustrittsfläche des Handgriffes können bei der Sterilisation angegriffen werden, so dass mit zunehmender Nutzungsdauer immer weniger Licht in den Lichtführungsstrang des Hakenelements eingekoppelt werden kann.

Es ist eine Aufgabe der vorliegenden Erfindung, einen beleuchteten medizinischen Retraktor bereitzustellen, der einen einfachen Aufbau aufweist, leicht zu handhaben ist und eine ausreichende Ausleuchtung eines Operationsfeldes erlaubt. Es wird betont, dass Ausführungsformen der Erfindung nicht alle oder auch nur einen einzigen dieser Vorteile aufweisen müssen.

Die vorstehende Aufgabe wird durch die Kombination der Merkmale des unabhängigen Anspruchs 1 gelöst. Bevorzugte Weiterbildungen finden sich in den abhängigen Ansprüchen.

Ausführungsformen eines medizinischen Retraktors weisen einen Griffabschnitt und einen Hakenabschnitt auf. Der Griffabschnitt ist zumindest abschnittsweise als Hohlkörper ausgebildet, der einen Hohlraum aufweist. Beispielsweise kann der Griffabschnitt vollständig entlang seiner Längsrichtung (Richtung seiner größten Erstreckung) als Hohlkörper ausgebildet sein. Eine den Hohlraum begrenzende Wandung des Griffabschnittes kann über die ganze Erstreckung des Hohlkörpers eine konstante Stärke aufweisen. Der Hakenabschnitt weist wenigstens einen Lichtleiter mit einer Lichteintrittsfläche und einer Lichtaustrittsfläche auf. Dabei sind der Griffabschnitt, der Hakenabschnitt und der Lichtleiter einstückig ausgebildet und damit dauerhaft starr miteinander verbunden. Der Griffabschnitt, der Hakenabschnitt und der Lichtleiter können nicht zerstörungsfrei voneinander getrennt werden. Die Lichteintrittsfläche des wenigstens einen Lichtleiters ist in dem Hohlraum angeordnet, der in dem Griffabschnitt ausgebildet ist, oder bündig zu einer Innenwand des in dem Griffabschnitt ausgebildeten Hohlraums angeordnet. Die Lichteintrittsfläche ist hin zu einem Inneren des Hohlraums orientiert, so dass im Inneren des Hohlraums erzeugtes Licht über die Lichteintrittsfläche in den Lichtleiter eintreten kann. Die Lichtaustrittsfläche des wenigstens einen Lichtleiters ist weg vom Griffabschnitt orientiert. Die Lichteintrittsfläche und Lichtaustrittsfläche können beispielsweise in entgegengesetzte Richtungen orientiert sein oder miteinander einen Winkel größer 60° einschließen. Der Lichtleiter als solches kann sich geradlinig oder gekrümmt erstrecken. Insbesondere kann er einem Verlauf des Haltehakens zumindest abschnittsweise folgen. Der Lichtleiter weist einen Querschnitt auf, der im Bereich der Lichtaustrittsfläche hin zur Lichtaustrittsfläche konstant ist oder zunimmt. Der Querschnitt des Lichtleiters kann auch über die ganze Erstreckung des Lichtleiters von der Lichteintrittsfläche hin zur Lichtaustrittsfläche konstant sein oder stetig zunehmen.

Indem der Griffabschnitt, der Hakenabschnitt und der Lichtleiter einstückig ausgebildet sind, entfällt eine Montage des Lichtleiters am Hakenabschnitt des Retraktors. Weiter besteht so nicht die Gefahr eines unbeabsichtigten Lösens des Lichtleiters vom Hakenabschnitt. Es ist auch möglich, den Griffabschnitt, den Hakenabschnitt und den Lichtleiter so auszubilden, dass keine Spalten zwischen dem Griffabschnitt, dem Hakenabschnitt und dem Lichtleiter verbleiben, was hinsichtlich Hygiene Vorteile bringt. Der Retraktor kann als Einmalteil ausgebildet sein.

Indem der Querschnitt des Lichtleiters im Bereich der Lichtaustrittsfläche hin zur Lichtaustrittsfläche konstant ist oder zunimmt, kommt es bei einem Austritt von Licht aus dem Lichtleiter nicht zu einer Bündelung, sondern zu einer Streuung, wodurch eine verglichen mit dem Stand der Technik große Fläche des Operationsfeldes ausgeleuchtet werden kann. Dabei ist zu beachten, dass der Hakenabschnitt des Retraktors im Einsatz häufig sehr nahe am Operationsfeld angeordnet ist. Gemäß einer Ausführungsform wird unter "dem Bereich der Lichtaustrittsfläche" der Abschnitt des Lichtleiters verstanden, der sich von der Lichtaustrittsfläche um höchstens 10% der größten Längserstreckung des Lichtleiters hin zur Lichteintrittsfläche des Lichtleiters erstreckt.

Indem der Griffabschnitt zumindest abschnittsweise als Hohlkörper ausgebildet ist, kann er mit geringem Materialeinsatz ausreichend dick ausgebildet werden, um gut gegriffen werden zu können. Weiter kann er so in seinem Inneren eine Lichtquelle und eine Energiequelle aufnehmen, die dann nicht mehr sterilisiert zu werden brauchen.

Der Retraktor kann beispielsweise als Roux-Haken, Langenbeck-Haken oder Fritsch-Haken ausgebildet sein.

Gemäß einer Ausführungsform ist zwischen dem Griffabschnitt, dem Hakenabschnitt und dem Lichtleiter kein Gelenk vorgesehen.

Gemäß einer Ausführungsform weist der Lichtleiter an seiner Lichtaustrittsfläche eine Zerstreuungslinse oder eine plane Fläche auf, die senkrecht zu einer Längsrichtung (Richtung größter Erstreckung) des Lichtleiters orientiert ist. Hierdurch kann eine Streuung des austretenden Lichtes erzielt werden.

Erfindungsgemäß weist der medizinischer Retraktor weiter einen Deckel auf, welcher den im Griffabschnitt ausgebildeten Hohlraum insbesondere flüssigkeitsdicht und weiter insbesondere luftdicht verschließt. Der Deckel kann beispielweise als Schraubverschluss, Bügelverschluss oder Bajonettverschluss ausgebildet sein, und eine separate Dichtung beispielsweise aus Kunststoff aufweisen. Auf diese Weise ist es möglich, in dem Hohlraum des Hohlkörpers einen unsterilen Körper aufzunehmen, und gegen Einflüsse von außen abzuschirmen. Es ist dann ausreichend, wenn der Retraktor außerhalb des Hohlraums des Hohlkörpers steril ist. Dies kann beispielsweise leicht dadurch erreicht werden, dass der Retraktor als steril einzeln verpacktes Einmalteil ausgebildet ist.

Erfindungsgemäß ist am Griffabschnitt wenigstens ein Haken ausgebildet, der bei verschlossenem Deckel mit dem Deckel in Eingriff kommt, und weist der Griffabschnitt wenigstens eine Sollbruchstelle auf, welche durch Abbrechen ein Öffnen des verschlossenen Deckels erlaubt. Gemäß einer Ausführungsform ist die Sollbruchstelle am Haken ausgebildet. Gemäß einer alternativen Ausführungsform ist die Sollbruchstelle benachbart zu einem am Griffabschnitt ausgebildeten Schraubgewinde ausgebildet. Gemäß einer Ausführungsform sind der Deckel und der wenigstens eine Haken so aufeinander abgestimmt, dass nur bei intaktem Haken ein sicheres Verschließen des Deckels möglich ist. Auf diese Weise kann sichergestellt werden, dass ein als Einmalteil ausgebildeter Retraktor nicht nochmals verwendet werden kann.

Gemäß einer Ausführungsform weist der Griffabschnitt in dem als Hohlkörper ausgebildeten Bereich eine Öffnung auf, welche eine den Hohlraum des Hohlkörpers umschließende Wand durchbricht, und ist diese Öffnung durch eine Abdeckung aus Elastomer und insbesondere Silikon flüssigkeitsdicht und insbesondere luftdicht verschlossen. Auf diese Weise ist es möglich, Druck auf einen im Hohlraum des Hohlkörpers aufgenommene Körper auszuüben, ohne dass ein Austausch von Stoffen zwischen dem Hohlraum und einer Außenseite des Retraktors erfolgt. Hierdurch wird beispielsweise eine Betätigung von im Inneren des Hohlkörpers aufgenommenen Druckschaltern ermöglicht. Gemäß einer Ausführungsform ist die Öffnung in Umfangsrichtung des Griffabschnitts angeordnet. Gemäß einer weiteren Ausführungsform ist die Öffnung an einer Stirnseite des Griffabschnitts und damit in Längsrichtung angeordnet. Gemäß einer Ausführungsform ist die Öffnung in einem den im Griffabschnitt ausgebildeten Hohlraum verschließenden Deckel angeordnet.

Gemäß einer Ausführungsform weist der Griffabschnitt an seiner Oberfläche wenigstens einen Bereich auf, der aus rutschhemmendem Material und insbesondere Elastomer gebildet und flächig mit dem Griffabschnitt verbunden ist. Dies ist vorteilhaft, da Retraktoren häufig in einem feuchten Umfeld eingesetzt werden, und sicher mit Handschuhen gehalten werden müssen. Gemäß einer weiteren Ausführungsform sind in dem Griffabschnitt eine oder mehrere Griffmulden oder ein oder mehrere die Reibung erhöhende insbesondere verrundete Vorsprünge ausgebildet.

Gemäß einer Ausführungsform weist der Griffabschnitt einen Anschlussbereich auf, der ausgebildet ist, an einem Haltesystem eines Operationstisches befestigt zu werden. In der Folge muss der Retraktor während eines chirurgischen Eingriffes nicht durchgehend gehalten werden, sondern kann zeitweise oder durchgehend fixiert werden. Der Anschlussbereich ist somit einstückig mit dem Griffabschnitt ausgebildet, und aus demselben Material wie der Griffabschnitt gebildet.

Gemäß einer Ausführungsform weist der Griffabschnitt wenigstens einen plattenförmigen Vorsprung auf, in welchem wenigstens eine Befestigungsöffnung ausgebildet ist, welche zusammen mit dem plattenförmigen Vorsprung den Anschlussbereich bildet.

Gemäß einer Ausführungsform ist der Anschlussbereich in Form eines Paars von plattenförmigen Vorsprüngen mit jeweils einer Befestigungsöffnung ausgebildet, wobei die plattenförmigen Vorsprünge in einer gemeinsamen Ebene liegen. Gemäß einer Ausführungsform sind die plattenförmigen Vorsprünge so angeordnet, dass sie sich bezüglich des Griffabschnitts gegenüber liegen, und der Hakenabschnitt mit jedem der plattenförmigen Vorsprünge den gleichen Winkel einschließt.

Gemäß einer Ausführungsform weist der wenigstens eine plattenförmige Vorsprung benachbart zum Griffabschnitt einen Radius von zwischen 0,8 cm und 1,5 cm auf.

Gemäß einer Ausführungsform ist der Anschlussbereich in Form eines Kupplungselements ausgebildet, welches sich geradlinig parallel zur Längserstreckung des Griffabschnitts erstreckt. Gemäß einer Ausführungsform ist das Kupplungselement auf der Seite des Griffabschnitts angeordnet, welche dem Hakenabschnitt abgewandt ist. Gemäß einer Ausführungsform weist das Kupplungselement einen trapezförmigen Querschnitt auf. Gemäß einer Ausführungsform sind mehrere Kupplungselemente vorgesehen, die sich bezüglich des Griffabschnitts paarweise gegenüber liegen.

Gemäß einer Ausführungsform ist der Anschlussbereich benachbart zum Hakenabschnitt am Griffabschnitt ausgebildet.

Gemäß einer Ausführungsform ist das Kupplungselement ein Vorsprung. Gemäß einer Alternativen Ausführungsform ist das Kupplungselement eine Vertiefung.

Gemäß einer Ausführungsform sind der Griffabschnitt und der Hakenabschnitt sowie der Lichtleiter aus dem gleichen Material gebildet. In der Folge kann der Retraktor besonders einfach und kostengünstig im Spritzgießverfahren hergestellt werden.

Gemäß einer alternativen Ausführungsform sind der Griffabschnitt und der Hakenabschnitt aus einem ersten Material gebildet, und ist der Lichtleiter aus einem von dem ersten Material verschiedenen zweiten Material gebildet, welches gemeinsam mit dem ersten Material koextrudiert ist. Somit müssen das erste Material und das zweite Material koextrudierbare Materialien sein. Der Griffabschnitt, der Hakenabschnitt und der Lichtleiter bilden so ein Koextrusionsprodukt. Die Verwendung unterschiedlicher Materialien erlaubt es, für den Lichtleiter ein Material zu verwenden, welches besonders gute lichtleitenden Eigenschaften aufweist, und für den Griffabschnitt und Hakenabschnitt ein Material zu verwenden, welches besonders steif und/oder bruchstabil ist. Durch die Koextrusion ist die Herstellung gleichwohl kostengünstig möglich.

Gemäß einer alternativen Ausführungsform sind der Griffabschnitt und der Hakenabschnitt aus einem Kunststoffmaterial gebildet, und ist der Lichtleiter ein aus einem von dem Kunststoffmaterial verschiedenen zweiten Material und insbesondere Quarzglas gebildetes Einlegeteil, welches durch Umspritzen mit Kunststoffmaterial dauerhaft flächig mit dem Kunststoffmaterial verbunden ist. Somit ist der Lichtleiter teilweise oder vollständig mit einem Kunststoffmaterial umspritzt. Die Verwendung unterschiedlicher Materialien erlaubt es, für den Lichtleiter ein Material zu verwenden, welches besonders gute lichtleitenden Eigenschaften aufweist, und für den Griffabschnitt und Hakenabschnitt ein Material zu verwenden, welches besonders steif und/oder bruchstabil ist. Durch das Umspritzen ist eine dauerhafte und sichere Verbindung des Lichtleiters mit dem Hakenteil möglich. Weiter ist es so sogar möglich, für den Lichtleiter ein Material zu verwenden, das bei Überlastung splittert, da durch das Umspritzen des Lichtleiters mit dem Kunststoffmaterial vermieden werden kann, dass Splitter in das Operationsfeld gelangen.

Gemäß einer Ausführungsform ist der Lichtleiter aus Polymethylmethacrylat, Polycarbonat, Polyetheretherketon oder Quarzglas gebildet. Diese Materialien weisen eine gute lichtleitende Eigenschaft auf, da an Grenzflächen Totalreflexion auftreten kann.

Gemäß einer Ausführungsform sind der Griffabschnitt und der Hakenabschnitt aus Polymethylmethacrylat, Polyetheretherketon oder Polycarbonat gebildet. Diese Materialien weisen eine gute Wärmeformbeständigkeit, optische Klarheit, hohe mechanische Belastbarkeit und Schlagzähheit auf. Zudem erlauben diese Materialien die Herstellung des Lichtleiters und Griffabschnitts und Hakenabschnitts aus demselben Material.

Gemäß einer Ausführungsform weist der im Griffabschnitt ausgebildete Hohlraum einen ovalen oder polygonen Innenquerschnitt auf. Dies erlaubt eine verdrehungssichere Anordnung eines entsprechend geformten Körpers im vom Hohlkörper umschlossenen Hohlraum. Sofern sich diese Form auch auf der Außenseite des Griffabschnitts wiederfindet, kann der Retraktor zudem verdrehungssicher gehalten werden.

Gemäß einer Ausführungsform weist der im Griffabschnitt ausgebildete Hohlraum eine Länge von zwischen 5 cm und 20 cm und insbesondere eine Länge von zwischen 7,5 cm und 15 cm auf. Gemäß einer Ausführungsform weist der im Griffabschnitt ausgebildete Hohlraum einen größten Innendurchmesser von zwischen 1 cm und 8 cm und insbesondere von zwischen 2 cm und 5 cm und weiter insbesondere 3 cm auf. Sofern sich diese Dimensionierung mit einem Zuschlag von höchstens einem Zentimeter auch auf der Außenseite des Griffabschnitts wiederfindet, kann der Retraktor bequem von Händen eines Benutzers gehalten werden.

Gemäß einer Ausführungsform weist der Lichtleiter einen runden oder ovalen oder rechteckigen oder trapezoiden oder dreieckigen Querschnitt auf.

Gemäß einer Ausführungsform schließen eine (Haupt-) Erstreckungsrichtung des Griffabschnitts und eine (Haupt-) Erstreckungsrichtung des Hakenabschnitts miteinander einen Winkel von zwischen 60° und 180° und insbesondere von zwischen 80° und 100° und weiter insbesondere von 90° ein.

Gemäß einer Ausführungsform weist der Hakenabschnitt eine Länge von zwischen 1 cm bis 40 cm und insbesondere von zwischen 10 cm bis 30 cm und weiter insbesondere von 20 cm auf.

Gemäß einer Ausführungsform weist der Hakenabschnitt eine Breite von zwischen 0,5 cm bis 10 cm und insbesondere von zwischen 1,5 cm bis 5 cm und weiter insbesondere von 4 cm auf.

Gemäß einer Ausführungsform weist der Lichtleiter eine Länge auf, welche zwischen 50 % und 100 % und insbesondere zwischen 60 % und 90 % der Länge des Haltehakens entspricht. Indem nur in dem Bereich des Haltehakens, der dem Griffabschnitt abgewandt ist, ein Lichtaustritt aus dem Lichtleiter erfolgt, können ungewünschte Blend- und Streueffekte unterdrückt werden.

Gemäß einer Ausführungsform weist der medizinische Retraktor weiter eine oder mehr als eine Lichtquelle auf, welche zusammen mit einer Energiequelle, insbesondere einer Batterie oder einem Akkumulator, in einem gemeinsamen Gehäuse angeordnet ist, welches Gehäuse eine oder mehr als eine Lichtaustrittsfläche aufweist, die optisch mit der wenigstens einen Lichtquelle verbunden ist. Dabei ist das die wenigstens eine Lichtquelle aufnehmende Gehäuse so in dem Griffabschnitt ausgebildeten Hohlraum anordenbar, dass die wenigstens eine Lichtaustrittsfläche des Gehäuses hin zu der Lichteintrittsfläche des wenigstens einen Lichtleiters ausgerichtet ist. Beispielsweise kann eine Form und Größe des die wenigstens eine Lichtquelle aufnehmenden Gehäuses so gewählt sein, dass das Gehäuse an einer Innenwand des im Griffabschnitt ausgebildeten Hohlraums flächig zur Anlage kommt. Bei der wenigstens einen Lichtquelle kann es sich beispielsweise um eine lichtemittierende Diode LED und insbesondere um eine Hochleistungs-LED oder um eine Laserdiode handeln. Die Verwendung einer Gasentladungs- oder Glühlampe ist alternativ möglich.

Gemäß einer Ausführungsform ist im Inneren des im Griffabschnitt ausgebildete Hohlraums wenigstens ein Haken ausgebildet, der das die Lichtquelle aufnehmende Gehäuse im Inneren des Griffabschnittes haltert, wenn das die Lichtquelle aufnehmende Gehäuse in dem Griffabschnitt angeordnet ist, wobei der Haken eine Sollbruchstelle aufweist, welche durch Abbrechen des wenigstens einen Haltehakens eine Entnahme des die Lichtquelle aufnehmenden Gehäuses aus dem Griffabschnitt erlaubt. Hierfür kann der Haltehaken die Lichtaustrittsfläche des Gehäuses beispielsweise in Richtung der Lichteintrittsfläche des Lichtleiters elastisch vorspannen und so eine gute Anlage der Lichtaustrittsfläche des Gehäuses an der Lichteintrittsfläche des Lichtleiters sicherstellen. Auf diese Weise kann sichergestellt werden, dass als Einmalartikel ausgebildete Retraktoren tatsächlich auch nur einmal verwendet werden können. Der Haken kann einstückig mit dem Griffabschnitt ausgebildet sein.

Gemäß einer Ausführungsform ist die Lichtaustrittsfläche des die Lichtquelle aufnehmenden Gehäuses komplementär zur Lichteintrittsfläche des wenigstens einen Lichtleiters ausgebildet. Dies erlaubt eine flächige Anlage der beiden Flächen aneinander und eine verlustarme Überleitung geführten Lichtes.

Gemäß einer Ausführungsform weist der Retraktor weiter einen Schalter auf, wobei der Schalter zwischen der Energiequelle und der Lichtquelle angeordnet und ausgebildet ist, die Lichtquelle wahlweise mit der Energiequelle zu verbinden. Schalter sind sehr kostengünstige und zuverlässige passive Bauelemente.

Gemäß einer alternativen Ausführungsform weist der Retraktor weiter eine Steuerung sowie einen Schalter auf, wobei die Steuerung mit der Energiequelle, dem Schalter und der Lichtquelle verbunden und ausgebildet ist, die Lichtquelle infolge einer Betätigung des Schalters zu steuern. Die Verwendung einer Steuerung erlaubt es, mehr als zwei Betriebszustände der Lichtquelle anzuwählen, ohne dass für eine Benutzereingabe eine Vielzahl von Schaltern erforderlich ist. Bei Verwendung einer Steuerung kann es sich bei dem Schalter insbesondere um einen Taster handeln.

Gemäß einer Ausführungsform ist der Schalter als Taster und insbesondere als Folientaster oder Sensortaster ausgebildet, und ist die Steuerung ausgebildet, die Lichtquelle infolge einer wiederholten Betätigung des Tasters nacheinander wenigstens zwischen den folgenden Betriebszuständen umzuschalten:
Lichtquelle wird mit reduzierter Leistung betrieben;
Lichtquelle wird mit maximaler Leistung betrieben;
Lichtquelle wird nicht betrieben.
Der Sensortaster kann beispielsweise nach dem Hall-Prinzip arbeiten oder als kapazitiver Taster oder Piezotaster ausgebildet sein.

Gemäß einer Ausführungsform entspricht die Strahlungsleistung der Lichtquelle bei reduzierter Leistung zwischen 25 % und 75 % der Strahlungsleistung der Lichtquelle bei maximaler Leistung.

Gemäß einer Ausführungsform weist der Griffabschnitt in dem als Hohlraum ausgebildeten Bereich eine Öffnung auf, welche eine den Hohlraum umschließende Wand durchbricht und durch eine Abdeckung aus Elastomer und insbesondere Silikon flüssigkeitsdicht und insbesondere luftdicht verschlossen ist. Dann ist das die Lichtquelle aufnehmende Gehäuse so in dem in dem Griffabschnitt ausgebildeten Hohlraum anordenbar ist, dass der Schalter benachbart zur Öffnung angeordnet ist. Auf diese Weise kann der Schalter über die Abdeckung aus Elastomer betätigt werden.

Gemäß einer Ausführungsform weist das die Lichtquelle aufnehmende Gehäuse einen ovalen oder polygonen Außenquerschnitt auf. Der Außenquerschnitt kann komplementär zu einem Innenquerschnitt des Hohlraums im Griffabschnitt ausgebildet sein. Alternativ können an der Innenwand des Hohlraums im Inneren des Griffabschnitts auch Vorsprünge zur Führung des Gehäuses ausgebildet sein. Auf diese Weise kann sichergestellt werden, dass das die Lichtquelle aufnehmende Gehäuse nur korrekt orientiert von dem Hohlraum im Griffabschnitt aufgenommen werden kann.

Gemäß einer Ausführungsform weist das die Lichtquelle aufnehmende Gehäuse eine Länge von zwischen 5 cm und 20 cm und insbesondere eine Länge von zwischen 7,5 cm und 15 cm auf.

Gemäß einer Ausführungsform weist das die Lichtquelle aufnehmende Gehäuse einen größten Außendurchmesser von zwischen 1 cm und 8 cm und insbesondere von zwischen 2 cm und 5 cm und weiter insbesondere 3 cm auf.

Gemäß einer Ausführungsform weist wenigstens die Lichtaustrittsfläche des wenigstens einen Lichtleiters eine Nanobeschichtung auf, welche einer Oberfläche der Lichtaustrittsfläche eine superhydrophobe oder superhydrophile Eigenschaft verleit. Gemäß einer Ausführungsform weist wenigstens der ganze Hakenabschnitt eine Nanobeschichtung auf, welche einer Oberfläche des ganzen Hakenabschnitts eine superhydrophobe oder superhydrophile Eigenschaft verleit. Dies erleichtert es, die Lichtaustrittsfläche frei von Verschmutzungen zu halten, so dass das im Lichtleiter geführte Licht möglichst wenig gedämpft wird.

Gemäß einer Ausführungsform weist wenigstens die Lichtaustrittsfläche des wenigstens einen Lichtleiters eine mikrostrukturierte Oberfläche auf, welche der Oberfläche der Lichtaustrittsfläche einen Lotuseffekt verleit. Gemäß einer Ausführungsform weist wenigstens der ganze Hakenabschnitt eine mikrostrukturierte Oberfläche auf, welche der Oberfläche des ganzen Hakenabschnitts einen Lotuseffekt verleit. Dies erleichtert es, die Lichtaustrittsfläche frei von Verschmutzungen zu halten, so dass das im Lichtleiter geführte Licht möglichst wenig gedämpft wird.

Ausführungsformen einer Beleuchtung für einen medizinischen Retraktor weisen eine Lichtquelle auf, welche zusammen mit einer Energiequelle, insbesondere einer Batterie oder einem Akkumulator, in einem gemeinsamen Gehäuse angeordnet sind, welches Gehäuse eine Lichtaustrittsfläche aufweist, die optisch mit der Lichtquelle verbunden ist. Weiter weist die Beleuchtung eine Steuerung sowie einen Schalter auf, wobei die Steuerung mit der Energiequelle, dem Schalter und der Lichtquelle verbunden und ausgebildet ist, die Lichtquelle infolge einer Betätigung des Schalters zu steuern. Der Schalter ist als Taster und insbesondere als Folientaster oder Sensortaster ausgebildet. Die Steuerung ist ausgebildet, die Lichtquelle infolge einer wiederholten Betätigung des Tasters nacheinander wenigstens zwischen den folgenden Betriebszuständen umzuschalten: Lichtquelle wird mit reduzierter Leistung betrieben; Lichtquelle wird mit maximaler Leistung betrieben; Lichtquelle wird nicht betrieben. Die Strahlungsleistung der Lichtquelle bei reduzierter Leistung entspricht zwischen 25 % und 75 % der Strahlungsleistung der Lichtquelle bei maximaler Leistung. Das die Lichtquelle aufnehmende Gehäuse kann einen ovalen oder polygonen Außenquerschnitt aufweisen. Das die Lichtquelle aufnehmende Gehäuse kann eine Länge von zwischen 5 cm und 20 cm und insbesondere eine Länge von zwischen 7,5 cm und 15 cm aufweisen. Das die Lichtquelle aufnehmende Gehäuse kann einen größten Außendurchmesser von zwischen 1 cm und 8 cm und insbesondere von zwischen 2 cm und 5 cm aufweisen.

In diesem Zusammenhang wird darauf hingewiesen, dass die in dieser Beschreibung und den Ansprüchen zur Aufzählung von Merkmalen verwendeten Begriffe "umfassen", "aufweisen", "beinhalten", "enthalten" und "mit", sowie deren grammatikalische Abwandlungen, generell als nichtabschließende Aufzählung von Merkmalen, wie z. B. Verfahrensschritten, Einrichtungen, Bereichen, Größen und dergleichen aufzufassen sind, und in keiner Weise das Vorhandensein anderer oder zusätzlicher Merkmale oder Gruppierungen von anderen oder zusätzlichen Merkmalen ausschließen.

Weitere Merkmale der Erfindung ergeben sich aus der nachfolgenden Beschreibung von Ausführungsbeispielen in Verbindung mit den Ansprüchen sowie den Figuren. In den Figuren werden gleiche bzw. ähnliche Elemente mit gleichen bzw. ähnlichen Bezugszeichen bezeichnet. Es wird darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung eines Ausführungsbeispiels der Erfindung wird auf die beiliegenden Figuren Bezug genommen, von denen
- Figur 1A: eine Seitenansicht eines medizinischen Retraktors gemäß einer Ausführungsform zeigt;
- Figur 1B: eine Rückansicht des medizinischen Retraktors gemäß der Ausführungsform aus Figur 1A zeigt;
- Figur 1C: eine Draufsicht des medizinischen Retraktors aus Figur 1A zeigt gemäß einer ersten Ausführungsform zeigt;
- Figur 1D: eine Draufsicht des medizinischen Retraktors aus Figur 1A zeigt gemäß einer zweiten Ausführungsform zeigt;
- Figur 2: einen schematischen Aufbau einer Beleuchtung für den medizinischen Retraktor gemäß der Ausführungsform aus Figur 1A zeigt;
- Figur 3A: eine Seitenansicht eines medizinischen Retraktors gemäß einer dritten Ausführungsform zeigt; und
- Figur 3B: eine Draufsicht des medizinischen Retraktors aus Figur 3A zeigt.

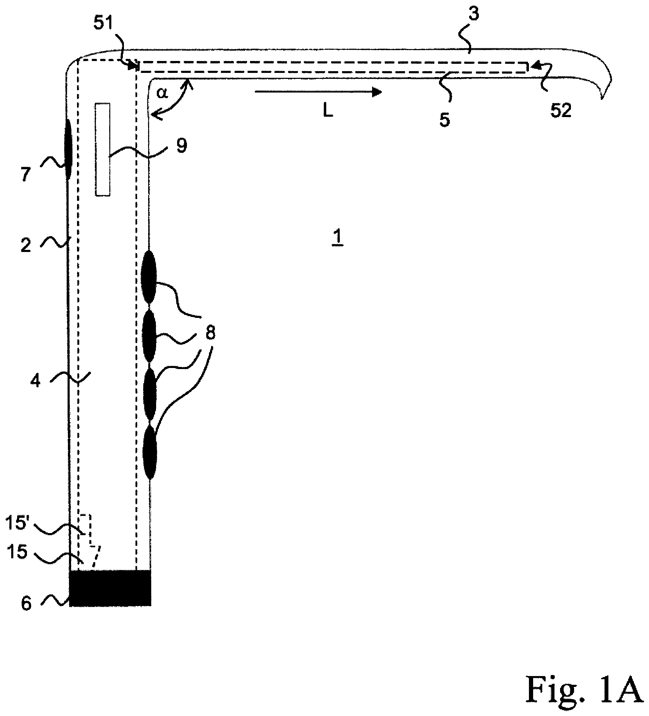

Im Folgenden wird unter Bezugnahme auf die Figuren 1A bis 1C ein medizinischer Retraktor 1 gemäß einer ersten Ausführungsform beschrieben. In den Figuren sind verdeckte Elemente mit gestrichelten Linien gezeichnet.

Der insgesamt in der Form eines Fritsch-Hakens ausgebildete medizinische Retraktor 1 weist einen Griffabschnitt 2 und einen Hakenabschnitt 3 auf, deren jeweilige Längserstreckung (Richtung größter Erstreckung) miteinander einen Winkel α von 90° einschließen.

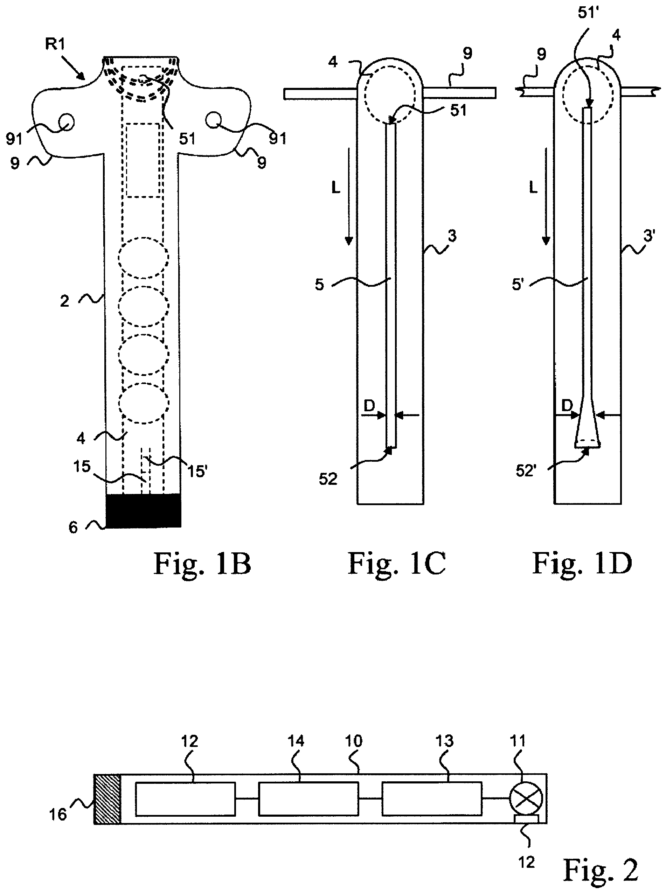

Der Griffabschnitt 2 ist als Hohlkörper mit ovalem Querschnitt ausgebildet, und umschließt einen Hohlraum 4 mit ebenfalls ovalem Querschnitt. Der Hohlraum 4 weist eine Länge von 15 cm, einen größten Durchmesser von 3,5 cm und einen kleinsten Durchmesser von 3 cm auf. Die den Hohlraum 4 begrenzende Wand des Griffabschnittes 2 weist über die ganze Erstreckung des Griffabschnitts 2 eine konstante Stärke von 2 mm auf. An seinem dem Hakenabschnitt 3 zugewandten Ende weist der Griffabschnitt 2 zwei symmetrisch ausgebildete Anschlussbereiche 9 mit Bohrungen 91 auf, die radial von dem Griffabschnitt vorstehen und an einem (nicht gezeigten) Haltesystem eines Operationstisches befestigt werden können. Dabei sind die Anschlussbereiche 9 durch plattenförmige Vorsprünge gebildet, in welche die Bohrungen 91 eingebracht sind. Dabei sind die plattenförmigen Vorsprünge in einer Ebene angeordnet, und liegen sich bezüglich des Griffabschnittes 2 gegenüber, wie aus Fig. 1C ersichtlich ist. An ihrer oberen, dem Hakenabschnitt 3 benachbarten Begrenzungskante weisen die plattenförmigen Vorsprünge jeweils einen Radius R1 von 1,5 cm auf. Ersichtlich sind die plattenförmigen Vorsprünge insgesamt ohne Ecken und damit abgerundet ausgebildet. Der Griffabschnitt 2 weist an seinem dem Hakenabschnitt 3 abgewandten Ende einen als Schnappverschluss ausgebildeten Deckel 6 auf, welcher den im Griffabschnitt 2 ausgebildeten Hohlraum 4 flüssigkeitsdicht und luftdicht verschließt. Um die Dichtheit bereitzustellen weist der Deckel 6 einen nicht gezeigten O-Ring aus Silikon auf. An seiner dem Hakenabschnitt 3 abgewandten Seite weist der Griffabschnitt 2 eine Öffnung auf, welche die den Hohlraum 4 umschließende Wand durchbricht, und durch eine Abdeckung aus Silikon 7 flüssigkeitsdicht und luftdicht verschlossen ist. Im Bereich des Deckels 6 ist im Griffabschnitt ein Schnapphaken 15 ausgebildet. Die Dimensionierung des Schnapphakens ist in Figur 1A stark überzeichnet. Schließlich weist der Griffabschnitt 2 an seiner dem Hakenabschnitt 3 zugewandten Seite an seiner Oberfläche vier voneinander in Längsrichtung des Griffabschnitts beabstandete ovale Bereiche 8 auf, die aus Elastomer gebildet und flächig mit dem Griffabschnitt 2 verbunden sind.

Der Hakenabschnitt 3 weist eine Länge von 20 cm und eine Breite von 4 cm sowie einen U-förmigen Querschnitt auf. Der Hakenabschnitt 3 weist einen Lichtleiter 5 mit einer Lichteintrittsfläche 51 und einer Lichtaustrittsfläche 52 auf, der einem Verlauf des Hakenabschnitts über 74 % seiner Längserstreckung folgt.

Die Lichteintrittsfläche 51 des Lichtleiters 5 ist bündig zu einer Innenwand des in dem Griffabschnitt 2 ausgebildeten Hohlraums 4 angeordnet und hin zu einem Inneren des Hohlraums 4 orientiert. Die Lichtaustrittsfläche 52 des Lichtleiters 5 ist in entgegengesetzte Richtung zur Lichteintrittsfläche 51 und damit weg vom Griffabschnitt 2 orientiert. Der Lichtleiter 5 weist einen runden Querschnitt mit einem Außendurchmesser D auf, welcher Außendurchmesser D über die ganze Längserstreckung L des Lichtleiters 5 einschließlich der Lichteintrittsfläche 51 und der Lichtaustrittsfläche 52 konstant ist.

Der Griffabschnitt 2, der Hakenabschnitt 3 und der Lichtleiter 5 sind in dieser ersten Ausführungsform als einstückiges Spritzgußteil aus Polymethylmethacrylat gebildet. Dabei ist der Retraktor 1 so geformt, dass keine Spalten zwischen dem Griffabschnitt 2, dem Hakenabschnitt 3 und dem Lichtleiter 5 bestehen. Die Oberfläche des Griffabschnitts 2, des Hakenabschnitts 3 und des Lichtleiters 5 sind mit einer Nanobeschichtung versehen, welche dieser Oberfläche eine superhydrophobe Eigenschaft verleit.

Im Folgenden wird unter Bezugnahme auf die Figuren 1A, 1B und 1D eine zweite Ausführungsform eines medizinischen Retraktors 1 beschrieben.

Da diese zweite Ausführungsform der vorstehend beschriebenen ersten Ausführungsform sehr ähnlich ist, wird im Folgenden nur auf Unterschiede eingegangen und ansonsten auf die Ausführungen zur ersten Ausführungsform verwiesen.

Die zweite Ausführungsform unterscheidet sich von der vorstehend beschriebenen Ausführungsform zum einen dadurch, dass der Lichtleiter 5' ein Einlegeteil aus Quarzglas ist, welches mit Ausnahme seiner Lichteintrittsfläche 51' und seiner Lichtaustrittsfläche 52' mit Polyetheretherketon umspritzt ist, aus welchem der Griffabschnitt 2 und der Hakenabschnitt 3' gebildet sind.

Weiter ragt die Lichteintrittsfläche 51' des Lichtleiters 5' in der zweiten Ausführungsform in den Hohlraum 4 hinein, der in dem Griffabschnitt 2 ausgebildet ist.

Zudem läuft der Lichtleiters 5', der in der zweiten Ausführungsform einen runden Querschnitt aufweist, entlang seiner Längsrichtung L hin zu der Lichtaustrittsfläche 52' auseinander, so dass sich sein Durchmesser hin zur Lichtaustrittsfläche 52' stetig vergrößert.

Schließlich ist an der Lichtaustrittsfläche 52' des Lichtleiters 5' in der zweiten Ausführungsform eine Zerstreuungslinse ausgebildet, deren Oberfläche so mikrostrukturiert ist, dass ein Lotuseffekt (geringe Benetzbarkeit der Oberfläche der Zerstreuungslinse, wie sie bei der Lotospflanze beobachtet werden kann), auftritt. Auf eine zusätzliche Nanobeschichtung des Griffabschnitts 2, des Hakenabschnitts 3 und des Lichtleiters 5 kann dann verzichtet werden.

Weiter wird der im Griffabschnitt 2 ausgebildete Hohlraum 4 gemäß der zweiten Ausführungsform durch einen als Schraubverschluss ausgebildeten Deckel 6 verschlossen. Hierfür weist der Griffabschnitt 2 im Bereich des Deckels 6 einen runden Querschnitt auf.

Es wird darauf hingewiesen, dass in Figur 1D die Anschlussbereiche 9 nur angeschnitten dargestellt sind.

Im Folgenden wird unter Bezugnahme auf die Figur 2 eine Ausführungsform einer Beleuchtung beschrieben, wie sie in den vorstehend beschriebenen medizinischen Retraktoren 1 verwendet werden kann.

Die Beleuchtung weist ein Gehäuse 10 aus lichtundurchlässigem Kunststoff auf, welches eine Lichtquelle in Form einer weißen Laserdiode 11, einen als Taster ausgebildeten Schalter 13, eine Steuerschaltung 14 und eine Energiequelle in Form einer Batterie 12 aufnimmt. Um ein Wechseln der Batterie 12 zu erlauben, ist das der Laserdiode 11 abgewandte ein Ende des Gehäuses 10 durch einen Schraubverschluss 16 verschlossen. Im Bereich der Laserdiode 11 weist das Gehäuse eine Lichtaustrittsfläche in Form eines Fensters 12 aus Quarzglas auf, welcher optisch mit einem Lichtauslass der Laserdiode 11 verbunden ist. Das Gehäuse weist einen leicht ovalen Querschnitt, eine Länge von 10 cm, einen größten Außendurchmesser von 3,45 cm und einen kleinsten Außendurchmesser von 2,95 cm auf. Damit ist der Querschnitt des Gehäuses 10 gut an den Querschnitt des Hohlraums 4 im Griffabschnitt 2 des vorstehend beschriebenen medizinischen Retraktors 1 angepasst.

Die Steuerschaltung 14 ist sowohl mit der Batterie 12, als auch mit dem Taster 13 und der Laserdiode 11 verbunden und ausgebildet, die Laserdiode 11 infolge einer wiederholten Betätigung des Tasters 13 nacheinander zwischen den folgenden Betriebszuständen umzuschalten:
Laserdiode 11 wird mit 50 % ihrer maximalen Leistung betrieben;
Laserdiode 11 wird mit ihrer maximaler Leistung betrieben; Laserdiode 11 wird nicht betrieben.

Während eines chirurgischen Eingriffes ist das Gehäuse 10 der Beleuchtung so in dem im Griffabschnitt 2 ausgebildeten Hohlraum 4 angeordnet, das Fenster 12 im Gehäuse 10 hin zu der Lichteintrittsfläche 51 des einen Lichtleiters 5 ausgerichtet ist, und der Taster 13 hinter der Abdeckung aus Silikon 7 des Griffabschnitts 2 angeordnet ist. Auf diese Weise kann der Schalter über die Abdeckung aus Silikon 7 betätigt werden.

In der gezeigten Ausführungsform ist eine Außenfläche des Fensters 12 an die Lichteintrittsfläche 51 angepasst und liegt bei in dem Hohlraum 4 des Griffabschnitts 2 angeordneter Beleuchtung flächig an der Lichteintrittsfläche 51 an.

Das Gehäuse 10 der Beleuchtung wird bei in dem Hohlraum 4 des Griffabschnitts 2 angeordneter Beleuchtung durch den Schnapphaken 15 in Richtung des Hackenabschnitts 3 vorgespannt. Der Schnapphaken 15 weist eine Sollbruchstelle 15' auf, welche durch Abbrechen des Schnapphakens 15 eine Entnahme der Beleuchtung aus dem Griffabschnitt 2 erlaubt.

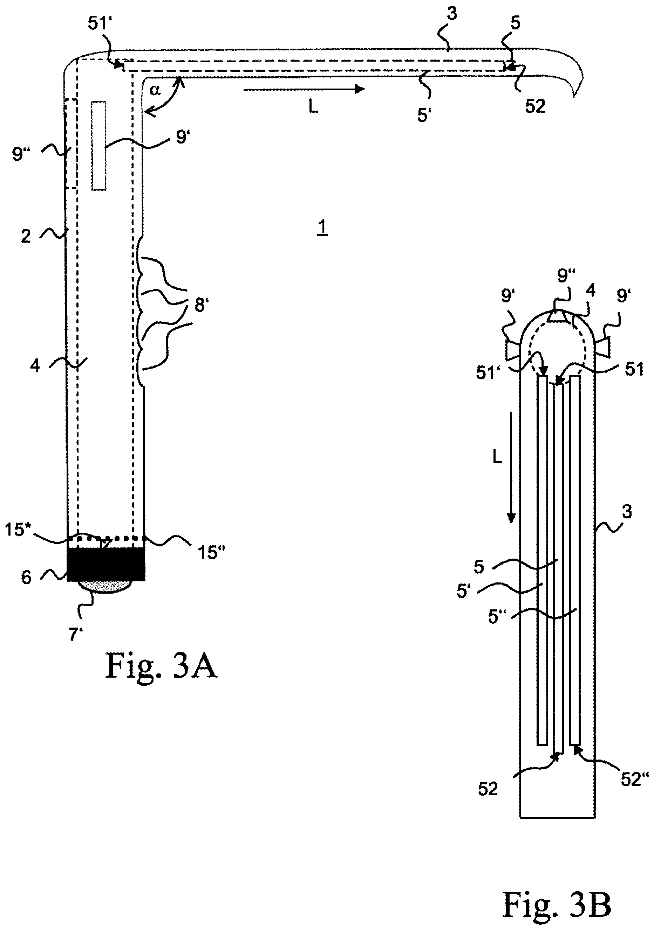

Im Folgenden wird unter Bezugnahme auf die Figuren 3A und 3B eine dritte Ausführungsform eines medizinischen Retraktors 1 beschrieben. Da diese Ausführungsform den vorstehend beschriebenen ersten und zweiten Ausführungsformen sehr ähnlich ist, wird nur auf Unterschiede zwischen diesen Ausführungsformen eingegangen und ansonsten auf das vorstehend Gesagte verwiesen.

Die dritte Ausführungsform unterscheidet sich von den vorstehend beschriebenen ersten und zweiten Ausführungsformen insbesondere dadurch, dass drei Lichtleiter 5, 5', 5" mit jeweils einer Lichteintrittsfläche 52, 52" und einer Lichtaustrittsfläche 51, 51' vorgesehen sind. Der besseren Übersichtlichkeit halber wurden nicht alle Lichteintrittsflächen und Lichtaustrittsflächen mit Bezugszeichen versehen.

Entsprechend weist auch die verwendete Beleuchtung eine entsprechende Anzahl von Lichtquellen in Form von Hochleistungs-LEDs auf. Die Beleuchtung ist in den Figuren jedoch nicht eigens gezeigt, da sie sich von der Beleuchtung der Figur 2 nur durch eine zweite und dritte Laserdiode und ein zugehöriges zweites und drittes Fenster unterscheidet.

Weiter unterscheidet sich die dritte Ausführungsform von den vorstehend beschriebenen ersten und zweiten Ausführungsformen dadurch, dass der Anschlussbereich in Form dreier Kupplungselemente 9', 9" ausgebildet, welche sich jeweils geradlinig parallel zur Längserstreckung des Griffabschnitts erstrecken und jeweils einen trapezförmigen Querschnitt aufweisen. Dabei sind zwei Kupplungselemente 9' so angeordnet, dass sie sich bezüglich des Griffabschnitts 2 gegenüber liegen. Das dritte Kupplungselement 9" liegt dem Hakenabschnitt 3 gegenüber. Zwei Kupplungselemente 9' sind als Vorsprünge, ein Kupplungselement 9" ist als Vertiefung ausgebildet.

Zusätzlich unterscheidet sich die dritte Ausführungsform von den vorstehend beschriebenen ersten und zweiten Ausführungsformen dadurch, dass am Griffabschnitt 2 ein Haken 15* ausgebildet ist, der bei verschlossenem Deckel 6 mit dem Deckel 6 in Eingriff kommt. Weiter weist der Griffabschnitt 2 benachbart zu diesem Haken 15* eine Sollbruchstelle 15" auf, welche durch Abbrechen ein Öffnen des verschlossenen Deckels 6 erlaubt. In der gezeigten Ausführungsform ist die Sollbruchstelle 15" sowohl dem Haken 15* als auch einem (nicht gezeigten) Gewinde für den Deckel 6 benachbart angeordnet.

Zudem unterscheidet sich die dritte Ausführungsform von den vorstehend beschriebenen ersten und zweiten Ausführungsformen dadurch, dass am Griffabschnitt 2 anstelle der aus Elastomer ausgebildeten ovalen Bereiche 8 mehrere die Reibung erhöhende Griffmulden und Vorsprünge 8" vorgesehen sind.

Schließlich unterscheidet sich die dritte Ausführungsform von der vorstehend beschriebenen ersten und zweiten Ausführungsform dadurch, dass der Schalter 7' an der dem Hakenabschnitt 3 abgewandten Stirnseite des Griffabschnitts 2 und damit im Deckel 6 angeordnet ist.

## Patentansprüche

1. Medizinischer Retraktor (1) aufweisend:
einen Griffabschnitt (2); und
einen Hakenabschnitt (3);
wobei der Griffabschnitt (2) zumindest abschnittsweise als einen Hohlraum (4) aufweisenden Hohlkörper ausgebildet ist,
wobei der Hakenabschnitt (3) wenigstens einen Lichtleiter (5) mit einer Lichteintrittsfläche und einer Lichtaustrittsfläche aufweist,
wobei die Lichteintrittsfläche (51) des wenigstens einen Lichtleiters (5) in dem in dem Griffabschnitt (2) ausgebildeten Hohlraum (4) angeordnet ist, oder bündig zu einer Innenwand des in dem Griffabschnitt (2) ausgebildeten Hohlraums (4) angeordnet ist, oder so angeordnet ist, dass sie hin zu dem in dem Griffabschnitt (2) ausgebildeten Hohlraum (4) orientiert ist; und
wobei die Lichtaustrittsfläche (52) des wenigstens einen Lichtleiters (5) weg vom Griffabschnitt (2) orientiert ist,
**dadurch gekennzeichnet, dass**
der medizinische Retraktor (1) weiter einen Deckel (6) aufweist, welcher den im Griffabschnitt (2) ausgebildeten Hohlraum (4) insbesondere flüssigkeitsdicht und weiter insbesondere luftdicht verschließt,
wobei am Griffabschnitt (2) wenigstens ein Haken (15*) ausgebildet ist, der bei verschlossenem Deckel (6) mit dem Deckel (6) in Eingriff kommt, wobei der Griffabschnitt wenigstens eine Sollbruchstelle (15") aufweist, welche durch Abbrechen des wenigstens einen Hakens (15*) ein Öffnen des verschlossenen Deckels erlaubt.

2. Medizinischer Retraktor (1) nach Anspruch 1, wobei die wenigstens eine Sollbruchstelle (15") am Haken (15*) ausgebildet ist.

3. Medizinischer Retraktor (1) nach Anspruch 1 oder 2,
wobei die Lichtaustrittsfläche (52) des wenigstens einen Lichtleiters (5) so ausgebildet ist, dass sie eine Streuung des austretenden Lichtes bewirkt; und/oder wobei die weg vom Griffabschnitt (2) orientierte Lichtaustrittsfläche (52) größer oder gleich einem Querschnitt (D) durch den Lichtleiter (5) im Bereich der Lichtaustrittsfläche (52) ist.

4. Medizinischer Retraktor (1) nach einem der Ansprüche 1 bis 3, wobei der Deckel (6) als Schraubverschluss oder als Bügelverschluss oder als Bajonettverschluss ausgebildet ist.

5. Medizinischer Retraktor (1) nach einem der Ansprüche 1 bis 4, wobei der Deckel (6) und der wenigstens eine Haken (15*) so aufeinander abgestimmt sind, dass nur bei intaktem Haken (15*) ein sicheres Verschließen des im Griffabschnitt (2) ausgebildeten Hohlraums (4) mittels des Deckels (6) möglich ist.

6. Medizinischer Refraktor (1) nach einem der Ansprüche 1 bis 5, wobei der Griffabschnitt (2) einen Anschlussbereich (9) aufweist, der ausgebildet ist, an einem Haltesystem eines Operationstisches befestigt zu werden, und insbesondere einstückig mit dem Griffabschnitt (2) ausgebildet ist.

7. Medizinischer Refraktor (1) nach Anspruch 6, wobei der Anschlussbereich in Form eines Paars von plattenförmigen Vorsprüngen mit jeweils einer Befestigungsöffnung ausgebildet ist, wobei die plattenförmigen Vorsprünge in einer gemeinsamen Ebene und sich bezüglich des Griffabschnitts (2) gegenüber liegen.

8. Medizinischer Retraktor (1) nach Anspruch 7, wobei der wenigstens eine plattenförmige Vorsprung benachbart zum Griffabschnitt einen Radius von zwischen 0,8 cm und 1,5 cm aufweist.

9. Medizinischer Retraktor (1) nach einem der Ansprüche 1 bis 8,
wobei der Lichtleiter (5) aus Polymethylmethacrylat, Polycarbonat, Polyetheretherketon oder Quarzglas gebildet ist; und/oder
wobei der Griffabschnitt (2) und der Hakenabschnitt (3) aus Polymethylmethacrylat, Polyetheretherketon oder Polycarbonat gebildet sind.

10. Medizinischer Refraktor (1) nach einem der Ansprüche 1 bis 9,
wobei der im Griffabschnitt (2) ausgebildete Hohlraum (4) eine Länge von zwischen 5 cm und 20 cm aufweist; und/oder
wobei der im Griffabschnitt (2) ausgebildete Hohlraum (4) einen größten Innendurchmesser von zwischen 1 cm und 8 cm oder von zwischen 2 cm und 5 cm aufweist; und/oder
wobei der Lichtleiter (5) einen runden oder ovalen oder rechteckigen oder trapezoiden oder dreieckigen Querschnitt aufweist.

11. Medizinischer Refraktor (1) nach einem der Ansprüche 1 bis 10,
wobei eine Erstreckungsrichtung des Griffabschnitts und eine Erstreckungsrichtung des Hakenabschnitts einen Winkel (α) von zwischen 60° und 180° oder von zwischen 80° und 100° oder von 90° einschließen; und/oder
wobei der Hakenabschnitt (3) eine Länge von zwischen 1 cm bis 40 cm oder von zwischen 10 cm bis 30 cm oder von 20 cm aufweist; und/oder
wobei der Hakenabschnitt (3) eine Breite von zwischen 0,5 cm bis 10 cm oder von zwischen 1,5 cm bis 5 cm oder von 4 cm aufweist.

12. Medizinischer Refraktor (1) nach einem der Ansprüche 1 bis 11,
weiter aufweisend eine Lichtquelle (11), welche zusammen mit einer Energiequelle (12) in einem gemeinsamen Gehäuse (10) angeordnet ist, welches Gehäuse (10) eine Lichtaustrittsfläche (12) aufweist, die optisch mit der Lichtquelle (11) verbunden ist;
wobei das Gehäuse (10) mit der Lichtquelle (11) so in dem in dem Griffabschnitt (2) ausgebildeten Hohlraum (4) anordenbar ist, dass die Lichtaustrittsfläche (12) des Gehäuses (10) hin zu der Lichteintrittsfläche (51) des wenigstens einen Lichtleiters (5) ausgerichtet ist.

13. Medizinischer Refraktor (1) nach Anspruch 12, wobei im Inneren des im Griffabschnitt (2) ausgebildeten Hohlraums (4) wenigstens ein Haken (15) ausgebildet ist, der das die Lichtquelle (11) aufnehmende Gehäuse (10) im Inneren des Griffabschnittes (2) haltert, wenn es in dem Griffabschnitt (2) angeordnet ist, wobei der Haken (15) eine Sollbruchstelle aufweist, welche durch Abbrechen des wenigstens einen Hakens (15) eine Entnahme des Gehäuses (10) aus dem Griffabschnitt (2) erlaubt.

14. Medizinischer Refraktor (1) nach einem der Ansprüche 12 oder 13,
wobei weiter eine Steuerung (14) sowie ein Taster (13) vorgesehen sind, welche Steuerung (14) zusammen mit der Lichtquelle (11) und der Energiequelle (12) in dem Gehäuse (10) aufgenommen ist, wobei die Steuerung (14) mit der Energiequelle (12), dem Taster (13) und der Lichtquelle (11) verbunden und ausgebildet ist, die Lichtquelle (11) infolge einer Betätigung des Tasters (13) zu steuern; und
wobei die Steuerung (14) ausgebildet ist, die Lichtquelle (11) infolge einer wiederholten Betätigung des Tasters nacheinander wenigstens zwischen den folgenden Betriebszuständen umzuschalten:
Lichtquelle (11) wird mit reduzierter Leistung betrieben;
Lichtquelle (11) wird mit maximaler Leistung betrieben;
Lichtquelle (11) wird nicht betrieben.

15. Medizinischer Retraktor (1) nach Anspruch 14,
wobei der Griffabschnitt (2) in dem als Hohlkörper ausgebildeten Bereich eine Öffnung aufweist, welche eine den Hohlraum (4) umschließende Wand durchbricht;
wobei die Öffnung durch eine Abdeckung (7) aus Elastomer und insbesondere Silikon flüssigkeitsdicht und insbesondere luftdicht verschlossen ist; und
wobei das die Lichtquelle (11) aufnehmende Gehäuse (10) so in dem in dem Griffabschnitt (2) ausgebildeten Hohlraum (4) anordenbar ist, dass der Taster (13) benachbart zur Öffnung angeordnet ist.
